# EUROPEAN PATENT APPLICATION

(11) **EP 1 891 897 A1**
(43) Date of publication of application: **27.02.2008**
(21) Application number: 06757025.9
(22) Date of filing: 06.06.2006
(51) Int. Cl.: A61B 5/145

(54) **SENSOR FOR MEASURING BIOLOGICAL COMPONENT**

(30) Priority: 07.06.2005 JP 2005166886
(71) Applicant: Omron Healthcare Co., Ltd., Kyoto 615-0084 (JP)
(72) Inventor: TOKITA, Muneo c/o OMRON HEALTHCARE CO., LTD., Ukyo-ku, Kyoto-shi Kyoto 6150084 (JP)
(74) Representative: Wilhelms . Kilian & Partner Patentanwälte
(86) International application number: PCT/JP2006/311285
(87) International publication number: WO 2006/132218

(57) **Abstract**

A biological component measuring sensor (10) comprising a plurality of light-receiving regions (21, 22) provided in a light-receiving element (20) for receiving light from an organism, and a plurality of waveguides (15, 16) provided in correspondence with the plurality of light-receiving regions (21, 22), each including an in-let side opening through which the light enters and an out-let side opening from which the light exits, and introducing the light to the plurality of light-receiving regions (21, 22), respectively. The plurality of waveguides (15, 16) are provided in a waveguide formation member (14) formed integrally, and the opening area of the in-let side opening of each of the waveguides (15, 16) is larger than the opening area of the out-let side opening. Consequently, the sensor can receive light from an organism more efficiently.

## Description

### TECHNICAL FIELD

The present invention relates to a biological component measuring sensor used to measure the concentration of a particular component included in a living tissue in a noninvasive manner using a spectroscopic approach.

### BACKGROUND ART

To measure the concentration of a particular component included in a living tissue as represented by blood or humor is very important to know the health conditions of a subject. The typical components to be measured include, for example, glucose, hemoglobin, oxyhemoglobin, neutral fat, cholesterol, albumin, uric acid, and the like included in the blood. For example, measuring the concentration of glucose included in the blood is very useful for early detection and treatment of diabetics. The concentration measurement of a variety of components other than those described above is carried out.

As described above, in the medical field, the measurement of concentration of particular components included in living tissues is frequently carried out. However, the employment of an invasive method of taking a sample of blood, humor or the like from a subject and analyzing it puts an enormous burden on the subject, and thus the method is not necessarily a preferable measurement method. Therefore, the methods that allow concentration measurement of particular components included in living tissues in a noninvasive manner have been researched elaborately, and one of such methods is a measurement method using a spectroscopic approach.

In the spectroscopic measurement method, the concentration measurement of a particular component included in a living body is carried out by sampling radiation light emitted from a living body or transmitted light transmitted through a living body or reflected light reflected on a living body, and detecting the spectrum of the sampled light. For example, in the case where a blood glucose concentration as described above is measured, the blood glucose concentration is measured by analyzing the spectrum in the near-infrared range or the mid-infrared range included in the radiation light radiated from the eardrum in the auditory meatus which is a part to be measured. As an apparatus that realizes this spectroscopic measurement method, a biological component measuring sensor is used.

The documents disclosing the aforementioned biological component measuring sensor includes, for example, Japanese National Publication No. 2001-503999 (Patent Document 1). The biological component measuring sensor disclosed in this publication is to measure a blood glucose concentration, in which light beams of two or more wavelengths in the mid-infrared range radiated from the eardrum are captured and a blood glucose concentration is measured based on these outputs. In the biological component measuring sensor disclosed in the aforementioned publication, since the blood glucose concentration is measured by capturing the radiation light radiated from the eardrum, there is no need for providing a light source separately in the apparatus, thereby simplifying the apparatus structure and enabling the apparatus to be manufactured cheaply.
Patent Document 1: Japanese National Publication No. 2001-503999

### DISCLOSURE OF THE INVENTION

### PROBLEMS TO BE SOLVED BY THE INVENTION

However, in the biological component measuring sensor disclosed in the aforementioned Japanese National Publication No. 2001-503999, the radiation light radiated from the eardrum is very weak, and it is very difficult to measure the blood glucose concentration accurately using this weak radiation light. In particular, in the biological component measuring sensor disclosed in the aforementioned publication, a waveguide installed in a probe inserted into the auditory meatus has a straight pipe shape, and two light-receiving regions are arranged in prescribed regions of a rear-end opening portion of the waveguide. Therefore, of the quantity of light passing through the waveguide, the quantity of light actually received at the light-receiving regions is only part of the whole quantity, and the efficient light reception is hardly carried out.

The present invention is therefore made to solve the aforementioned problem. An object of the present invention is to realize a biological component measuring sensor capable of receiving light from a living body efficiently so that the concentration measurement of a particular component included in a living tissue can be carried out with high accuracy.

### MEANS FOR SOLVING THE PROBLEMS

A biological component measuring sensor based on the present invention spectroscopically measures a concentration of a particular component included in a living tissue in a noninvasive manner and includes a plurality of light-receiving regions and a plurality of waveguides. Each of the aforementioned plurality of light-receiving regions is provided in light-receiving means for receiving light from a living body. The aforementioned plurality of waveguides each include an inlet-side opening portion where the light enters and an outlet-side opening portion where the light exists and are provided respectively corresponding to the plurality of light-receiving regions to introduce the light to the respective plurality of light-receiving regions. The aforementioned plurality of waveguides are provided in an interior of a waveguide formation member as integrally formed. In each of the aforementioned plurality of waveguides, an opening area of the inlet-side opening portion is formed to be larger than an opening area of the outlet-side opening portion.

Here, "light from a living body" as mentioned above includes radiation light emitted from a living body as well as transmitted light and reflected light of light radiating from a light source to a living body. Furthermore, "waveguide formation member as integrally formed" as mentioned above means one formed of a plurality of waveguides in the interior thereof as one component and one formed by combining and integrating a plurality of parts each having a waveguide formed therein with each other, and is meant to exclude a case where a plurality of parts each having a waveguide formed therein are separately arranged without being combined with each other.

Because of such a configuration, light from a living body branches off and enters each of a plurality of waveguides, and the light entering each waveguide is condensed along the shape of the waveguide and enters the light-receiving region in this condensed state. In other words, in the inlet-side opening portion of the waveguide, the opening area is larger so that a larger amount of light enters the waveguide, while in the outlet-side opening portion of the waveguide, the area is narrower than that of the inlet-side opening portion, so that the quantity of light per unit area is increased. Therefore, the quantity of light entering each of a plurality of light-receiving regions is increased, the light-receiving efficiency in the light-receiving region is improved, and the concentration measurement of particular components included in living tissues can be performed accurately. In addition, since a plurality of waveguides are provided in the waveguide formation member as integrally formed, the positioning of the light-receiving means and the waveguide becomes easier, and in addition, it becomes possible to reduce the size of the apparatus.

In the biological component measuring based on the present invention as described above, each of the aforementioned plurality of waveguides is preferably formed to have its opening area gradually reduced from the inlet-side opening portion to the outlet-side opening portion.

Because of such a configuration, light entering each of a plurality of waveguides can be condensed at the outlet-side opening portion with the required minimum number of times of reflection on the wall surface of the waveguide, so that absorption or scattering of light at the time of reflection is prevented, thereby realizing higher light-receiving efficiency.

In the biological component measuring sensor based on the present invention as described above, in each of the aforementioned plurality of waveguides, an opening shape in the inlet-side opening portion and an opening shape in the outlet-side opening portion may be formed to be different from each other.

Because of such a configuration, in the inlet-side of the waveguide, the shape of the inlet-side opening portion can be selected so that light enters the waveguide to a maximum extent, while in the outlet-side of the waveguide, the shape of the outlet-side opening portion can be selected according to the shape of the light-receiving region so that the condensed light enters the light-receiving region without loss. Therefore, the higher light-receiving efficiency can be realized.

### EFFECTS OF THE INVENTION

In accordance with the present invention, a biological component measuring sensor capable of receiving light from a living body efficiently is achieved, so that the concentration measurement of particular components included in living tissues can be performed with high accuracy.

### BRIEF DESCRIPTION OF THE DRAWINGS

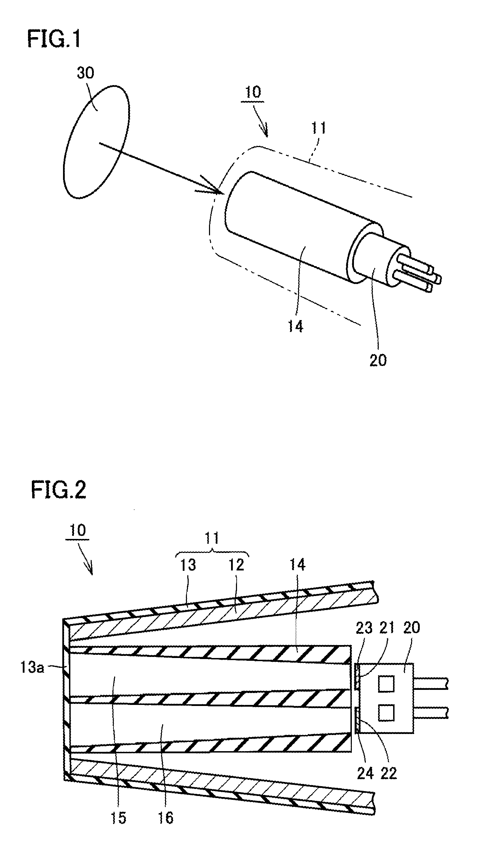

Fig. 1 is a view schematically showing a usage state of a biological component measuring sensor in an embodiment of the present invention.
Fig. 2 is a schematic cross-sectional view of the biological component measuring sensor shown in Fig. 1.

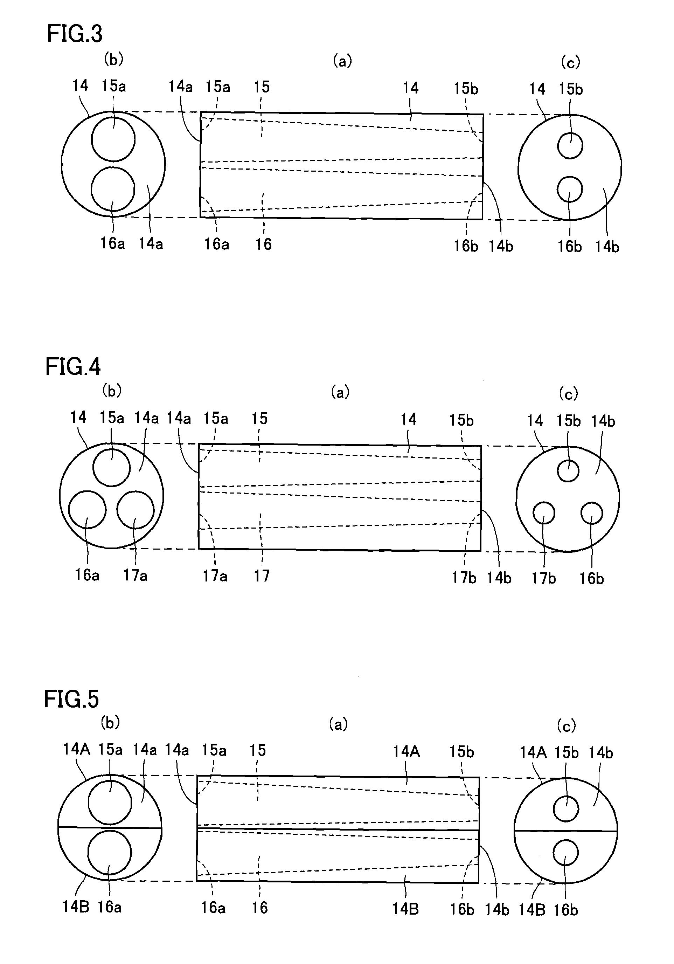

Fig. 3 is a view showing the shape of a waveguide formation member shown in Fig. 1 and Fig. 2, in which (a) is a side view of the waveguide formation member, (b) is a view showing the shape of an inlet-side end face of the waveguide formation member, and (c) is a view showing the shape of an outlet-side end face of the waveguide formation member.
Fig. 4 is a view showing a modification of the waveguide formation member of the biological component measuring sensor in the embodiment of the present invention, in which (a) is a side view of the waveguide formation member, (b) is a view showing the shape of the inlet-side end face of the waveguide formation member, and (c) is a view showing the shape of the outlet-side end face of the waveguide formation member.
Fig. 5 is a view showing another modification of the waveguide formation member of the biological component measuring sensor in the embodiment of the present invention, in which (a) is a side view of the waveguide formation member, (b) is a view showing the shape of the inlet-side end face of the waveguide formation member, and (c) is a view showing the shape of the outlet-side end face of the waveguide formation member.

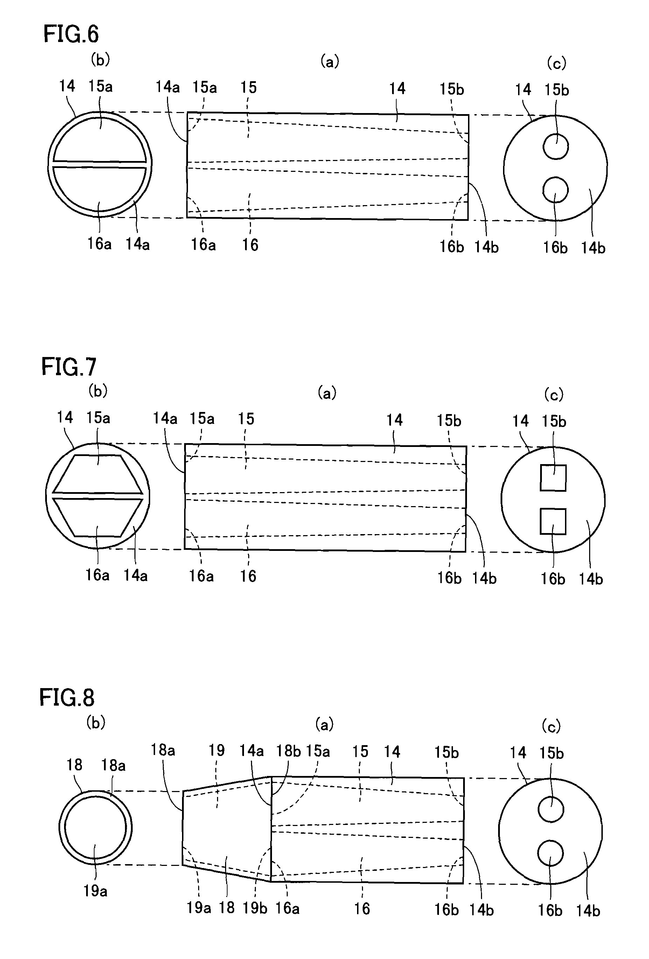

Fig. 6 is a view showing yet another modification of the waveguide formation member of the biological component measuring sensor in the embodiment of the present invention, in which (a) is a side view of the waveguide formation member, (b) is a view showing the shape of the inlet-side end face of the waveguide formation member, and (c) is a view showing the shape of the outlet-side end face of the waveguide formation member.
Fig. 7 is a view showing a further modification of the waveguide formation member of the biological component measuring sensor in the embodiment of the present invention, in which (a) is a side view of the waveguide formation member, (b) is a view showing the shape of the inlet-side end face of the waveguide formation member, and (c) is a view showing the shape of the outlet-side end face of the waveguide formation member.
Fig. 8 is a view showing a still further modification of the waveguide formation member of the biological component measuring sensor in the embodiment of the present invention, in which (a) is a side view of the waveguide formation member, (b) is a view showing the shape of the inlet-side end face of the waveguide formation member, and (c) is a view showing the shape of the outlet-side end face of the waveguide formation member.

### DESCRIPTION OF THE REFERENCE SIGNS

10 biological component measuring sensor, 11 probe portion, 12 protective casing, 13 dustproof film, 13a dustproof window, 14, 18 waveguide formation member, 14A, 14B member, 14a, 18a front face, 14b, 18b back face, 15, 16, 17, 19 waveguide, 15a, 16a, 17a, 19a inlet-side opening portion, 15b, 16b, 17b, 19b outlet-side opening portion, 20 light-receiving element, 21, 22 light-receiving region, 23, 24 filter, 30 eardrum (part to be measured)

### BEST MODES FOR CARRYING OUT THE INVENTION

In the following, an embodiment of the present invention will be described in detail with reference to the figures. It is noted that in the embodiment illustrated below, a description will be made by way of illustration to a case where the present invention is applied to a glucometer for measuring a blood glucose concentration by putting a probe portion containing a biological component measuring sensor into the auditory meatus, and detecting the spectra of wavelengths in two ranges of mid-infrared radiation radiated from the eardrum using the biological component measuring sensor.

Fig. 1 is a view schematically showing a usage state of a biological component measuring sensor in an embodiment of the present invention, and Fig. 2 is a schematic cross-sectional view of the biological component measuring sensor shown in Fig. 1. In addition, Fig. 3 is a view showing the shape of a waveguide formation member shown in Fig. 1 and Fig. 2, in which (a) is a side view of the waveguide formation member, (b) is a view showing the shape of an inlet-side end face of the waveguide formation member, and (c) is a view showing the shape of an outlet-side end face of the waveguide formation member.

As shown in Fig. 1, a biological component measuring sensor 10 in the present embodiment is included in a detection end of a glucometer to detect weak radiation light radiated from an eardrum 30 as a part to be measured, which is positioned in the auditory meatus. In measurement, as shown in Fig. 1, a probe portion 11 which is the detection end is inserted in the auditory meatus, and the front face (tip end face) of the inserted probe portion 11 is opposed to eardrum 30. In this state, radiation light radiated from eardrum 30 is introduced from the front face of probe portion 11 into probe portion 11.

As shown in Fig. 1 and Fig. 2, biological component measuring sensor 10 is mainly configured with a waveguide formation member 14 arranged in the interior of probe portion 11 formed of a protective casing 12 and a dustproof film 13 and a light-receiving element 20 as light-receiving means.

The above-noted protective casing 12 is formed of a tubular member having a front opening. Dustproof film 13 is attached to protective casing 12 to close the front opening of protective casing 12, and in particular, the part closing the front opening of protective casing 12 functions as a dustproof window 13a. Dustproof film 13 is a film for preventing intrusion of dust into the interior of probe portion 11, and a thin film of, for example, plastic, glass, silicon, or germanium is used for this dustproof film 13. In the present embodiment, a polyethylene film is used so that radiation light radiated from eardrum 30 is transmitted well.

As shown in Fig. 2, waveguide formation member 14 is formed of an integrally formed member having two waveguides 15, 16 in the interior thereof and is arranged such that the front face thereof faces dustproof window 13a of probe portion 11. Waveguide formation member 14 is formed, for example, of a resin material, a metal material or the like, and an inner wall surface defining waveguides 15, 16 formed in the interior thereof is mirror-finished. A variety of methods can be adopted as the method of mirror finish, and, for example, gold plating and deposition of gold, aluminum or the like are suitable.

At the back of waveguide formation member 14 in the interior of probe portion 11, light-receiving element 20 is arranged. Light-receiving element 20 is an element photoelectrically converting an optical signal received at the light-receiving region as described later into an electrical signal. As light-receiving element 20, for example, an element formed of two photodiodes on a single semiconductor substrate may be used or two elements each formed of one photodiode on a single semiconductor substrate may be used. On the main surface of light-receiving element 20, two light-receiving regions 21, 22 are provided. These light-receiving regions 21, 22 are regions receiving light from a living body.

On the surfaces of light-receiving regions 21, 22, filters 23, 24 are respectively affixed. Filters 23, 24 are spectroscopic means for transmitting only light having a wavelength in a particular range and preventing transmittance of light having a wavelength in the other ranges. In the present embodiment, used as filter 23 is a fileter transmitting the mid-infrared radiation with wavelengths of 9 µm-10 µm dependent on a blood glucose concentration, and used as filter 24 is a filter transmitting the mid-infrared radiation with wavelengths of 8 µm-9 µm independent of a blood glucose concentration. It is noted that although, in the present embodiment, a filter is used as spectroscopic means by way of illustration, a diffraction grating, a prism, or the like may be used otherwise.

As shown in Fig. 2, waveguides 15, 16 formed in the interior of waveguide formation member 14 extend toward in the front-to-back direction of probe portion 11, and have respective one opening portions facing the above-noted dustproof window 13a and the respective other opening portions facing light-receiving regions 21, 22 of light-receiving element 20 through filters 23, 24, respectively. As shown in Fig. 3(a) to Fig. 3(c), the aforementioned one opening portions are provided on a front face 14a of waveguide formation member 14 and are inlet-side opening portions 15a, 16a where radiation light radiated from eardrum 30 enters. On the other hand, the aforementioned other opening portions are provided on a back face 14b of waveguide formation member 14 and are outlet-side opening portions 15b, 16b where the aforementioned radiation light passing through waveguides 15, 16 exits.

As shown in Fig. 2 and Fig. 3(a) to Fig. 3(c), in waveguide formation member 14 of biological component measuring sensor 10 in the present embodiment, the opening areas of the aforementioned inlet-side opening portions 15a, 16a are formed to be larger than the opening areas of the aforementioned outlet-side opening portions 15b, 16b. In addition, each of waveguides 15, 16 is formed to have its opening area gradually reduced from the aforementioned inlet-side opening portions 15a, 16a to the aforementioned outlet-side opening portions 15b, 16b. Specifically, as an example, assume that, for example, the axial length of waveguide formation member 14 is 30 mm, inlet-side opening portions 15a, 16a are formed in a circular shape each having a diameter of 3 mm, and outlet-side opening portions 15b, 16b are formed in a circular shape each having a diameter of 1 mm.

Because of a configuration as described above, radiation light radiated from the eardrum branches off and enters each of two waveguides 15, 16, and the radiation light entering respective waveguides 15, 16 is condensed along the shape of waveguides 15, 16 and applied to filters 23, 24 in this condensed state. The radiation light applied to filters 23, 24 is separated into the mid-infrared radiations of wavelengths in two ranges at filters 23, 24, and only the separated mid-infrared radiations of wavelengths in the respective ranges enter the respective light-receiving regions 21, 22 of light-receiving element 20. Then, the light received at light-receiving element 20 is photoelectrically converted and output, so that the spectrum is detected based on this output signal in the glucometer main body and the blood glucose concentration is determined.

As described above, in biological component measuring apparatus 10 in the present embodiment, in inlet-side opening portions 15a, 16a of waveguides 15, 16, the opening areas thereof are larger so that a larger amount of light enters waveguides 15, 16, while in outlet-side opening portions 15b, 16b of waveguides 15, 16, the areas thereof are narrower than those of inlet-side opening portions 15a, 16a, so that the quantity of light per unit area is increased. Therefore, the quantity of light entering each of two light-receiving regions 21, 22 is increased and the light-receiving efficiency in light-receiving regions 21, 22 is improved. As a result, measurement of a blood glucose concentration included in a living tissue can be performed accurately.

In addition, since the opening areas are gradually reduced from inlet-side opening portions 15a, 16a to outlet-side opening portions 15b, 16b, light entering the respective two waveguides 15, 16 can be condensed in outlet-side opening portions 15b, 16b with the required minimum number of times of reflection on the wall surfaces of waveguides 15, 16. Therefore, absorption or scattering of light associated with reflection can be minimized and an optical loss is decreased, resulting in higher light-receiving efficiency. Therefore, measurement of a blood glucose concentration included in a living tissue can be performed accurately.

Moreover, the provision of two waveguides 15, 16 in waveguide formation member 14 as integrally formed facilitates the positioning of light-receiving regions 21, 22 of light-receiving element 20 and waveguides 15, 16, and also contributes to the size reduction of the apparatus.

In the foregoing, the description has been made by way of illustration to the case where the present invention is applied to the biological component measuring sensor incorporated in a glucometer measuring a blood glucose concentration by detecting the spectra of wavelengths in two ranges of mid-infrared radiation. However, the present invention is also applicable to a biological component measuring sensor incorporated in a measuring apparatus detecting any other biological component, for example, is applicable to one using mid-infrared radiation as well as one using near-infrared radiation or one using visible light. Furthermore, the components to be detected include, in addition to glucose included in the blood as described above, hemoglobin, oxyhemoglobin, neutral fat, cholesterol, albumin, uric acid, and the like.

When the components to be detected are varied, the configuration of the biological component measuring sensor needs to be modified in various manners. When the component to be detected is glucose as described above, the configuration of the biological component measuring sensor is also susceptible to a variety of modifications. In the following, examples of them will be described.

Fig. 4 to Fig. 8 are views showing modifications of the waveguide formation member of the biological component measuring sensor in the present embodiment, in each of which (a) is a side view showing the waveguide formation member, (b) is a view showing the shape of the inlet-side end face of the waveguide formation member, and (c) is a view showing the outlet-side end face of the waveguide formation member. In the following, referring to these figures, the modifications of the waveguide formation member of the biological component measuring sensor in the present embodiment will be described.

In a modification shown in Fig. 4(a) to Fig. 4(c), three waveguides 15, 16, 17 are formed in waveguide formation member 14. These three waveguides 15, 16, 17 are provided corresponding to three light-receiving regions provided in a not-shown light receiving element. Light entering waveguides 15, 16, 17 from inlet-side opening portions 15a, 16a, 17a provided on front face 14a of waveguide formation member 14 passes through waveguides 15, 16, 17, is condensed and exists at outlet-side opening portions 15b, 16b, 17b provided on back face 14b of waveguide formation member 14, and is then applied to and received at three light-receiving regions of a not-shown light-receiving element.

In the biological component measuring sensor performing concentration measurement of a particular component included in a living tissue, detection of spectra of wavelengths in three or more ranges is sometimes required, depending on a component to be detected. In addition, in some cases, detection of spectra of wavelengths in three or more ranges may be required in order to remove interference components. In such cases, the number of waveguides corresponding to the number of spectra of wavelengths of ranges to be detected may be formed in the interior of waveguide formation member 14 as integrally formed. Fig. 4(a) to Fig. 4(c) as described above show a modification in a case where the spectra of wavelengths in three ranges are supposed to be detected. However, depending on circumstances, the number of waveguides may be increased to four, five, or more.

A modification shown in Fig. 5(a) to Fig. 5(c) is formed by dividing waveguide formation member 14 into two members 14A, 14B in the direction parallel to the axial center and combining them by adhesion or the like for integration. Waveguides 15, 16 are respectively provided in members 14A, 14B. Then, on front face 14a of members 14A, 14B, inlet-side opening portions 15a, 16a of waveguides 15, 16 are respectively provided, and on back face 14b of members 14A, 14B, outlet-side opening portions 15b, 16b of waveguides 15, 16 are respectively provided.

In this manner, in the case where the waveguide formation member is formed by combining and integrating a plurality of members each having a waveguide formed therein with each other, the effect similar to the effect in the present embodiment as described above is also achieved. The advantage of combining and integrating a plurality of members with each other is for example as follows. When the waveguide formation member is fabricated by cutting a metal or molding a resin, shaping is difficult by such processing, and the employment of this configuration makes fabrication easier even in such a case.

In a modification shown in Fig. 6(a) to Fig. 6(c) and a modification shown in Fig. 7(a) to Fig. 7(c), the opening shape of inlet-side opening portions 15a, 16a of waveguides 15, 16 and the opening shape of outlet -side opening portions 15b, 16b of waveguides 15, 16 are different from each other. Specifically, in the modification shown in Fig. 6(a) to Fig. 6(c), the opening shape of inlet-side opening portions 15a, 16a is semicircular and the opening shape of outlet-side opening portions 15b, 16b is circular. On the other hand, in the modification shown in Fig. 7(a) to Fig. 7(c), the opening shape of inlet-side opening portions 15a, 16a is trapezoidal and the opening shape of outlet-side opening portions 15b, 16b is rectangular.

In this manner, in the case where the inlet-side opening portion and the outlet-side opening portion of the waveguide have different opening shapes, advantageously, at the inlet side of the waveguide, the shape of the inlet-side opening portion can be selected so that light enters the waveguide to a maximum extent, while at the outlet side of the waveguide, the shape of the outlet-side opening portion can be selected according to the shape of the light-receiving region so that the condensed light enters the light-receiving region without loss. Therefore, the higher light-receiving efficiency can be realized.

In a modification shown in Fig. 8(a) to Fig. 8(c), a waveguide formation member 18 of a separate member is connected in front of waveguide formation member 14 described in the foregoing embodiment which is shrunken in the axial direction. Waveguide formation member 18 has one waveguide 19 in the interior thereof and is provided with an inlet-side opening portion 19a of waveguide 19 on a front face 18a thereof and an outlet-side opening portion 19b of waveguide 19 on a back face 18b thereof, respectively. Waveguide formation member 18 and waveguide formation member 14 are connected to each other so that back face 18b and front face 14a are in contact with each other. Thus, each of waveguides 15, 16 is in communication with waveguide 19. In waveguide formation member 18, the opening area of inlet-side opening portion 19a provided on front face 18a thereof is formed smaller than the opening area of outlet-side opening portion 19b provided on back face 18b thereof As a result, waveguide formation member 18 has a tapered shape to the front face side from the back face side.

In such a configuration, the shape of the tip end portion of the biological component measuring sensor can be tapered, so that the shape of the probe portion of the measuring apparatus can be tapered, accordingly. Therefore, by applying the biological component measuring sensor having such a configuration to a measuring apparatus in which measurement has to be performed by putting the probe portion into a part narrower as getting deeper, such as an auditory meatus, the probe portion as a detection end can be formed in such a shape that can be easily put into the meatus, and the tip end of the probe portion can be brought closer to a part to be measured. Therefore, high-accuracy measurement can be performed.

In the embodiment and modifications thereof as described above, the description has been made by way of illustration to the biological component measuring sensor receiving radiation light radiated from a part to be measured of a living body. However, the present invention is also applicable to a biological component measuring sensor receiving transmitted light or reflected light of light applied from a light source to a part to be measured of a living body and photoelectrically converting the same, as a matter of course.

Furthermore, in the embodiment and modifications thereof as described above, the description has been made by way of illustration, assuming that a part to be measured of a living body is an eardrum. However, a part to be measured is not limited thereto and may be various parts of a living body.

In this manner, the foregoing embodiment as disclosed herein is illustrative rather than limitative in all respects. The technical scope of the present invention is defined by the claims, and it is intended that equivalents to the claims and all modifications within the scope are embraced.

## Claims

1. A biological component measuring sensor for spectroscopically measuring a concentration of a particular component included in a living tissue in a noninvasive manner, comprising:
a plurality of light-receiving regions provided in light-receiving means for receiving light from a living body; and
a plurality of waveguides, each including an inlet-side opening portion where said light enters and an outlet-side opening portion where said light exists, provided respectively corresponding to said plurality of light-receiving regions to introduce said light to respective said plurality of light-receiving regions, wherein
said plurality of waveguides are provided in an interior of a waveguide formation member as integrally formed, and
in each of said plurality of waveguides, an opening area of said inlet-side opening portion is formed to be larger than an opening area of said outlet-side opening portion.

2. The biological component measuring sensor according to claim 1, wherein each of said plurality of waveguides is formed to have its opening area gradually reduced from said inlet-side opening portion to said outlet-side opening portion.

3. The biological component measuring sensor according to claim 1, wherein in each of said plurality of waveguides, an opening shape in said inlet-side opening portion and an opening shape in said outlet-side opening portion are different from each other.
